# NEW EUROPEAN PATENT SPECIFICATION

(11) **EP 1 417 972 B2**
(45) Date of publication and mention of the opposition decision: **22.08.2018**
(45) Mention of the grant of the patent: 10.04.2013
(21) Application number: 03104219.5
(22) Date of filing: 07.12.1998
(51) Int. Cl.: A61K 38/29, A61K 47/10, A61K 9/08, A61K 47/26

(54) **Stabilized teriparatide solutions**
Teriparatidhaltige stabilisierte Lösungen
Solutions stabilisées de Tériparatide

(30) Priority: 09.12.1997 US 69075 P
(43) Date of publication of application: 12.05.2004
(62) Divisional of application: 98123225.9
(73) Proprietor: Eli Lilly & Company, Indianapolis, IN 46285 (US)
(72) Inventor: Chang, Chin-Ming, Fishers, 46038 (US); Havel, Henry A, Indianapolis 46220 (US)
(74) Representative: Marshall, Cameron John

(56) References cited:
- EP-A- 0 302 772
- EP-A- 0 619 119
- WO-A-91/06564
- WO-A-95/17207
- WO-A1-97/14429
- WO-A2-94/08613
- JP-A- S6 360 940
- US-A- 5 578 567
- DATABASE WPI Week 8817 Derwent Publications Ltd., London, GB; AN 88-115396 [17] XP002102409 & JP 63 060940 A (TOYO JOZO) 17 March 1988 (1988-03-17)
- CHEMICAL ABSTRACTS, vol. 111, no. 26, 25 December 1989 (1989-12-25) Columbus, Ohio, US; abstract no. 239514, XP002105393 & JP 01 016799 A (TOYO JOZO) 20 January 1989 (1989-01-20)
- KIMMEL D.B. ET AL: 'The effect of recombinant human (1-84) or synthetic human (1-34) parathyroid hormone on then skeleton of adult osteopenic ovariectomized rats' ENDOCRINOLLOGY vol. 132, no. 4, 1993, pages 1577 - 1584
- BONTEMPO J.A.: 'Development of Biopharmaceutical Parenteral Dosage Forms', 1997, MARCEL DEKKER, INC., NEW YORK pages 91 - 142
- REEVE J. ET AL: 'Calcified Tissue Research', vol. 21, 1976 pages 467 - 477
- TONG X-K. ET AL: 'Intersectin can regulate the Ras/MAP kinase pathway independent of its role in endocytosis' JOURNAL OF BIOLOGICAL CHEMISTRY vol. 275, no. 38, 2000, page 29894
- JOHN WANG Y-C. ET AL: 'Parenteral formulations of proteins and peptides: Stability and Stabilizers' JOURNAL OF PARENTERAL SCIENCE AND TECHNOLOGY vol. 42, 1988, pages S3 - S26

### SEQUENCE LISTING

<110> Eli Lilly and Company
<120> STABILIZED TERIPARATIDE SOLUTIONS
<130> 3797.16WO01
<140> NEW FILING
   <141> 1998-12-08
<150> 60/069,075
   <151> 1997-12-09
<160> 2
<170> Patentln Ver. 2.0
<210> 1
   <211> 84
   <212> PRT
   <213> Homo sapiens
<400> 1
   Ser Val Ser Glu Ile Gln Leu Met His Asn Leu Gly Lys His Leu Asn
   1 5 10 15
   Ser Met Glu Arg Val Glu Trp Leu Arg Lys Lys Leu Gln Asp Val His
   20 25 30
   Asn Phe Val Ala Leu Gly Ala Pro Leu Ala Pro Arg Asp Ala Gly Ser
   35 40 45
   Gln Arg Pro Arg Lys Lys Glu Asp Asn Val Leu Val Glu Ser His Glu
   50 55 60
   Lys Ser Leu Gly Glu Ala Asp Lys Ala Asn Val Asp Val Leu Thr Lys
   65 70 75 80
   Ala Lys Ser Gln
<210> 2
   <211> 34
   <212> PRT
   <213> Homo sapiens
<400> 2
   Ser Val Ser Glu Ile Gln Leu Met His Asn Leu Gly Lys His Leu Asn
   1 5 10 15
   Ser Met Glu Arg Val Glu Trp Leu Arg Lys Lys Leu Gln Asp Val His
   20 25 30
   Asn Phe

### SEQUENCE LISTING

<110> Eli Lilly and Company
<120> STABILIZED TERIPARATIDE SOLUTIONS
<130> 3797.16WO01
<140> NEW FILING
   <141> 1998-12-08
<150> 60/069,075
   <151> 1997-12-09
<160> 2
<170> Patentln Ver. 2.0
<210> 1
   <211> 84
   <212> PRT
   <213> Homo sapiens
<400> 1
   Ser Val Ser Glu Ile Gln Leu Met His Asn Leu Gly Lys His Leu Asn
   1 5 10 15
   Ser Met Glu Arg Val Glu Trp Leu Arg Lys Lys Leu Gln Asp Val His
   20 25 30
   Asn Phe Val Ala Leu Gly Ala Pro Leu Ala Pro Arg Asp Ala Gly Ser
   35 40 45
   Gln Arg Pro Arg Lys Lys Glu Asp Asn Val Leu Val Glu Ser His Glu
   50 55 60
   Lys Ser Leu Gly Glu Ala Asp Lys Ala Asn Val Asp Val Leu Thr Lys
   65 70 75 80
   Ala Lys Ser Gln
<210> 2
   <211> 34
   <212> PRT
   <213> Homo sapiens
<400> 2
   Ser Val Ser Glu Ile Gln Leu Met His Asn Leu Gly Lys His Leu Asn
   1 5 10 15
   Ser Met Glu Arg Val Glu Trp Leu Arg Lys Lys Leu Gln Asp Val His
   20 25 30
   Asn Phe

## Description

### TECHNICAL FIELD

This invention relates to pharmaceutical compositions containing a parathyroid hormone. More particularly, the invention relates to teriparatide, PTH(1-34), stabilized solution formulations

### BACKGROUND OF THE INVENTION

Parathyroid hormone (PTH) is a secreted, 84 amino acid product of the mammalian parathyroid gland that controls serum calcium levels through its action on various tissues, including bone. Studies in humans with certain forms of PTH have demonstrated an anabolic effect on bone, and have prompted significant interest in its use for the treatment of osteoporosis and related bone disorders.

Using the N-terminal 34 amino acids of the bovine and human hormone for example, which by all published accounts are deemed biologically equivalent to the full length hormone, it has been demonstrated in humans that parathyroid hormone enhances bone growth particularly when administered in pulsatile fashion by the subcutaneous route. A slightly different form of PTH, human PTH(1-38) has shown similar results.

PTH preparations have been reconstituted from fresh or lyophilized hormone, and incorporate various forms of carrier, excipient and vehicle. Most are prepared in water-based vehicles such as saline, or water acidified typically with acetic acid to solubilize the hormone. The majority of reported formulations also incorporate albumin as a stabilizer (see for example Reeve at al., Br. Med. J., 1980, 280:6228; Reeve at al., Lancet, 1976,1:1035; Reeve at al., Calcif. Tissue Res., 1976, 21:469; Hodsman et al., Bone Miner; 1990, 9(2):137; Tsai et al., J. Clin. Endocrinol Metab., 1989,69(5):1024; Isaac et al., Horm. Metab. Res., 1980, 12(9):487; Law et al., J. Clin Invest. 1983, 72(3):1106; and Hulter, J. Clin Hypertens, 1986, 2(4):360). Other reported formulations have incorporated an excipient such as mannitol, which is present either with the lyophilized hormone or in the reconstitution vehicle. Formulations representative of those employed for human studies include a human PTH(1-34) (SEQ ID NO: 2) preparation consisting, upon reconstitution, of mannitol, heat inactivated human serum albumin, and caproic acid (a protease inhibitor) as absorption enhancer (see Reeve at al., 1976, Calcif. Tissue Res., 21, Suppl., 469-477); a human PTH(1-38) preparation reconstituted into a saline vehicle (see Hodsman et al., 1991, 14(1), 67-83); and a bovine PTH(1-34) preparation in aqueous vehicle pH adjusted with acetic acid and containing albumin. There is also an International Reference preparation which for human PTH (1-84) (SEQ ID NO: 1) consists of 100 ng of hormone ampouled with 250 µg human serum albumin and 1.25 mg lactose (1981), and for bovine PTH (1-84) consists of 10 µg lyophilized hormone in 0.01M acetic acid and 0.1% w/v mannitol (see Martindale, The Extra Pharmacoepia, The Pharmaceutical Press, London, 29th Edition, 1989 at p. 1338).

A recent attempt at improving the stability for the lyophilized preparation of h-PTH(1-34) (SEQ ID NO: 2) is reported in EP 619 119 with a combination of sugar and sodium chloride. Also U.S. Pat. No. 5,496,801 describes a freeze-dried composition for the natural hormone, PTH(1-84), containing mannitol as an excipient and a citrate source as a non-volatile buffering agent.

Commercial exploitation of parathyroid hormone requires the development of a formulation that is acceptable in terms of storage stability and ease of preparation. Because it is a protein and thus far more labile than the traditionally small molecular weight drugs, however, the formulation of parathyroid hormone presents challenges not commonly encountered by the pharmaceutical industry. Furthermore, like other proteins that have been formulated successfully, PTH is particularly sensitive to oxidation, deamidation and hydrolysis, and requires that its N-terminal and C-terminal sequences remain intact in order to preserve bioactivity.

It is an object of the present invention to provide a pharmaceutically useful PTH preparation, particularly one comprising, as active ingredient, teriparatide, PTH(1-34) (SEQ ID NO: 2).

### SUMMARY OF THE INVENTION

The present invention provides a pharmaceutical composition in the form of a stabilized solution containing a parathyroid hormone (PTH) in a therapeutically effective amount. The solution is storage stable and, in sterile form, may be stored in vials or cartridges ready for parenteral administration in human patients. The advantages of the present solution is the elimination of the need for lyophilization.

Therefore the invention provides :

A process for preparing a pharmaceutical composition in the form of a sterile solution ready for parenteral administration, said process comprising: mixing human PTH(1-34), a buffering agent and an excipient to form an aqueous solution containing PTH in a concentration range from 25mg/mL to 1000mg/mL, which is then sterile-fiftered and filled into a vial or cartridge for use, wherein the excipient comprises a polyol stabilising agent, and wherein said composition further comprises a parenterally acceptable preservative.
- A pharmaceutical composition in the form of a stabilized solution, comprising: (a) a therapeutically effective amount of human PTH(1-34); (b) an effective amount of a polyol stabilizing agent; (c) a buffering agent in an amount sufficient to maintain the pH of the composition within a range of about 3-7; (d) a parenterally acceptable preservative; and (e) the balance being water.

### DETAILED DESCRIPTION

The invention relates to parathyroid hormone solutions that exhibit storage stability in terms of hormone composition and activity.

The hormone is human PTH(1-34) (SEQ ID NO: 2) also known as teriparatide. The hormone may be obtained by known recombinant or synthetic methods, such as described in U.S. Pat. No. 4,086,196.

The stabilizing agent incorporated into the solution or composition includes a polyol which includes a saccharide, preferably a monosaccharide or disaccharide, e.g., glucose, trehalose, raffinose, or sucrose; a sugar alcohol such as, for example, mannitol, sorbitol or inositol, and a polyhydric alcohol such as glycerine or propylene glycol or mixtures thereof. A preferred polyol is mannitol or propylene glycol. The concentration of polyol may range from about 1 to about 20 wt-%, preferably about 3 to 10 wt-% of the total solution.

The buffering agent employed in the solution or composition of the present invention may be any acid or salt combination which is pharmaceutically acceptable and capable of maintaining the aqueous solution at a pH range of 3 to 7, preferably 3-6. Useful buffering systems are, for example, acetate, tartrate or citrate sources. Preferred buffer systems are acetate or tartrate sources, most preferred is an acetate source. The concentration of buffer may be in the range of about 2 mM to about 500 mM, preferably about 2 mM to 100 mM.

The stabilized solution or composition of the present invention may also include a parenterally acceptable preservative. Such preservatives include, for example, cresols, benzyl alcohol, phenol, benzalkonium chloride, benzethonium chloride, chlorobutanol, phenylethyl alcohol, methyl paraben, propyl paraben, thimerosal and phenylmercuric nitrate and acetate. A preferred preservative is m-cresol or benzyl alcohol; most preferred is m-cresol. The amount of preservative employed may range from about 0.1 to about 2 wt-%, preferably about 0.3 to about 1.0 wt-% of the total solution.

Thus, the present invention has provided, for example, a stabilized teriparatide solution containing mannitol, acetate and m-cresol with a predicted shelf-life of over 13 months at 5°C.

The PTH solution and composition of the present invention incorporate PTH in a medically effective amount, a term used with reference to amounts useful either therapeutically or in medical diagnosis. The particular amount or parathyroid hormone incorporated in the preparation can be pre-determined based on the type of PTH selected and on the intended end-use of the preparation. In one application, the preparations are exploited for therapeutic purposes, and particularly for the treatment of osteoporosis. Osteoporosis therapy entails administration of the reconstituted preparation by injection, desirably subcutaneous injection, in unit doses that reflect the prescribed treatment regimen but are, by way of example, for human PTH(1-34) (SEQ ID NO: 2), within the range from 25 mg PTH/mL of injected solution to 1000 mg/mL of injected solution per patient, with injection volumes being desirably from 0.02 to 1.3 mL. Accordingly, the purified PTH is desirably incorporated with the buffering agent and excipient to form an aqueous solution containing PTH in a concentration range from 25 mg/mL to 1000 mg/mL, preferably 100 mg/mL to 500 mg/mL, which is then sterile-filtered and filled into a vial or cartridge for use.

Once the preparation is obtained as an aqueous solution containing desired amounts and concentrations of the buffering agent, excipient and PTH, individual vials are filled with the solution to the desired volume. The advantage of the present invention is that the above solution may be prepared with sterile water without the need to undergo a freeze-drying process.

In addition to their therapeutic use, the present PTH composition can be formulated and administered to aid in medical diagnosis, and particularly to assist in establishing the diagnosis of hypoparathyroidism and pseudohypoparathymidism in hypocalcemic patients. Except for the dose of PTH, the composition of the PTH preparation will remain as described herein for therapeutic use. An intravenously infused, single dose of human PTH(1-34) (SEQ ID NO: 2) that is equal to 200 International Units of PTH activity is appropriate for this diagnostic purpose. Diagnosis is then made by determining the effect of administered PTH or urinary cAMP levels, with cAMP elevation being indicative of the hypoparathyroidism condition, rather than its pseudoform.

The examples which follow are illustrative of the invention and are not intended to be limiting.

### EXAMPLES

### Example 1

0.1 mg rhPTH (1-34) (SEQ ID NO: 2), 50 mg mannitol, 2.5 mg m-cresol, 0.52 mg acetic acid and 0.12 mg sodium acetate were mixed into a solution with 1 ml of distilled water.

### Example 2

0.25 mg rhPTH (1-34) (SEQ ID NO: 2), 45.4 mg mannitol, 3 mg m-cresol, 0.41 mg acetic acid and 0.1 mg sodium acetate were mixed into a solution with 1 ml of distilled water.

The formulations of the present invention, Examples 1 and 2 were compared to solutions containing no stabilizer, 0.9% NaCl, 20 mM acetate and 10 mM acetate as primary stabilizer. The stability was measured by determining the amount in % of rhPTH (1-34) (SEQ ID NO: 2) remaining after a certain time. The measurement was made by HPLC. The results are shown in Tables 1 and 2.

**Table 1**

| Effect of Primary Stabilizer on Chemical Stability of rhPTH (1-34) at 50°C | | | | |
|---|---|---|---|---|
| | Water | 0.9% NaCl | 20 mM acetate | 10 mM acetate |
| Time, days | % Remaining | | | |
| Initial | 100 | 100 | 100 | 100 |
| 7 | 74 | 81 | 84 | 80 |
| 14 | 55 | 58 | 67 | 71 |

**Table 2**

| Comparison of Stability of rhPTH (1-34) at 30°C | | | | |
|---|---|---|---|---|
| | 20 mM acetate | 10 mM acetate | Example 1 | Example 2 |
| Time, days | % Remaining | | | |
| Initial | 100 | 100 | 100 | 100 |
| 7 | 96 | 94 | 100 | - |
| 14 | 94 | 92 | 96 | 100 |
| 21 | 90 | 93 | 97 | - |
| 30 | -- | 81 | 96 | 96 |

### Example 3 (for reference)

The following experiment was carried out to show that lyophilized powder formulations prepared from stabilized solutions of the present invention are more stable than a control which was prepared from PTH(1-34) and mannitol alone.

A control solution and solutions for samples A through O were prepared as previously described with the ingredients and concentrations shown in Table 3. The solutions were then freeze-dried and the resulting lyophilized powder formulations were stored at 40°C for a one month period. The amount of PTH(1-34) retaining in each sample was then measured by HPLC. The results are shown in Table 3.

**Table 3**

| Stability of PTH(1-34) Lyophilized Formulations at 40°C for One Month | | | | | | |
|---|---|---|---|---|---|---|
| Sample | PTH(1-34) mg/mL | Bulking Agent | Bulking Agent Conc. mg/mL | Buffer | Buffer. Conc. mM | % PTH Remaining |
| Control | 0.2 | mannitol | 40 | - | -- | 78 |
| A | 0.5 | mannitol | 30 | acetate | 5 | 90 |
| B | 0.5 | glycine | 30 | acetate | 5 | 98 |
| C | 0.5 | sucrose | 30 | acetate | 5 | 98 |
| D | 0.5 | trehalose | 30 | acetate | 5 | 97 |
| E | 0.5 | raffinose | 30 | acetate | 5 | 99 |
| F | 0.75 | mannitol | 30 | tartrate | 15 | 95 |
| G | 1.5 | sucrose & mannitol | 5/25 | tartrate | 5 | 99 |
| H | 0.75 | sucrose & mannitol | 5/25 | tartrate | 15 | 99 |
| I | 1.5 | mannitol | 30 | tartrate | 5 | 96 |
| J | 1.5 | sucrose | 30 | tartrate | 15 | 100 |
| K | 1.5 | mannitol | 30 | tartrate | 15 | 99 |
| L | 0.75 | sucrose | 30 | tartrate | 15 | 100 |
| M | 0.75 | sucrose | 30 | tartrate | 5 | 100 |
| N | 1.5 | sucrose & mannitol | 5/25 | tartrate | 15 | 99 |
| O | 1.5 | sucrose & mannitol | 5/25 | acetate | 5 | 91* |
| *the stability at 2 months was 96% | | | | | | |

## Claims

1. A process for preparing a pharmaceutical composition in the form of a sterile solution ready for parenteral administration, said process comprising: mixing human PTH(1-34), a buffering agent and an excipient to form an aqueous solution containing PTH in a concentration range from 25µg/mL to 1000µg/mL, which is then sterile-filtered and filled into a vial or cartridge for use, wherein the excipient comprises a polyol stabilising agent, and wherein said composition further comprises a parenterally acceptable preservative.

2. A process according to claim 1 wherein said buffering agent is selected from citrate, tartrate or acetate.

3. A process according to claim 2 wherein said buffering agent is acetate.

4. A process according to any one of claims 1-3 wherein the concentration of said buffering agent in said composition is in the range of 2mM to 500mM.

5. A process according to any one of claims 1-4 wherein said buffering agent maintains a pH from 3 to 6.

6. A process according to any one of claims 1-5 wherein said polyol stabilising agent is a saccharide, a sugar alcohol such as mannitol, or a polyhydric alcohol.

7. A process according to claim 6 wherein said polyol stabilising agent is present in an amount of 1-20 wt-% of the composition.

8. A process according to any preceding claim wherein said preservative is m-cresol or benzoyl alcohol.

9. A process according to any one of claims 1-8 wherein said preservative is present in an amount of 0.1-2 wt-% of the composition.

10. A process according to any one of claims 1-9 wherein the concentration of said PTH(1-34) is in the range 100µg/mL to 500µg/mL.

11. A pharmaceutical composition in the form of a stabilized solution, comprising: (a) a therapeutically effective amount of human PTH(1-34); (b) an effective amount of a polyol stabilizing agent; (c) a buffering agent in an amount sufficient to maintain the pH of the composition within a range of about 3-7; (d) a parenterally acceptable preservative; and (e) the balance being water.

## Patentansprüche

1. Verfahren zur Herstellung einer pharmazeutischen Zusammensetzung in Form einer sterilen Lösung, die für eine parenterale Verabreichung bereit ist, wobei das Verfahren ein Vermischen von humanem PTH(1-34), eines Puffers und eines Hilfsstoffs umfasst, wobei eine wässrige Lösung hergestellt wird, die PTH in einer Konzentration in einem Bereich von 25 µg/ml bis 1000 µg/ml enthält, wobei die Lösung anschließend sterilfiltriert und in eine Ampulle oder Patrone zur Verwendung gefüllt wird, wobei der Hilfsstoff ein Polyol-Stabilisierungsmittel umfasst, und wobei die Zusammensetzung ferner ein parenteral akzeptables Konservierungsmittel umfasst.

2. Verfahren nach Anspruch 1, wobei der Puffer aus Citrat, Tartrat oder Acetat ausgewählt ist.

3. Verfahren nach Anspruch 2, wobei der Puffer Acetat ist.

4. Verfahren nach einem der Ansprüche 1 bis 3, wobei die Konzentration des Puffers in der Zusammensetzung in einem Bereich von 2 mM bis 500 mM liegt.

5. Verfahren nach einem der Ansprüche 1 bis 4, wobei der Puffer einen pH-Wert von 3 bis 6 aufrechterhält.

6. Verfahren nach einem der Ansprüche 1 bis 5, wobei das Polyol-Stabilisierungsmittel ein Saccharid, ein Zuckeralkohol wie Mannit oder ein mehrwertiger Alkohol ist.

7. Verfahren nach Anspruch 6, wobei das Polyol-Stabilisierungsmittel in einer Menge von 1 bis 20 Gew.-%, bezogen auf die Zusammensetzung, vorhanden ist.

8. Verfahren nach einem vorhergehenden Anspruch, wobei das Konservierungsmittel m-Kresol oder Benzylalkohol ist.

9. Verfahren nach einem der Ansprüche 1 bis 8, wobei das Konservierungsmittel in einer Menge von 0,1 bis 2 Gew.-%, bezogen auf die Zusammensetzung, vorhanden ist.

10. Verfahren nach einem der Ansprüche 1 bis 9, wobei die Konzentration des PTH(1-34) in einem Bereich von 100 µg/ml bis 500 µg/ml liegt.

11. Pharmazeutische Zusammensetzung in Form einer stabilisierten Lösung, die a) eine therapeutisch wirksame Menge von humanem PTH(1-34); b) eine wirksame Menge eines Polyol-Stabilisierungsmittels; c) einen Puffer in einer Menge, die ausreicht, um den pH-Wert der Zusammensetzung in einem Bereich von etwa 3 bis 7 zu halten; d) ein parenteral akzeptables Konservierungsmittel und e) zum Rest Wasser umfasst.

## Revendications

1. Procédé de préparation d'une composition pharmaceutique se présentant sous la forme d'une solution stérile prête pour l'administration par voie parentérale, ledit procédé comprenant : le mélange de la PTH(1-34) humaine, d'un agent tampon et d'un excipient pour former une solution aqueuse contenant la PTH en une plage de concentrations de 25 µg/mL à 1 000 µg/mL, qui est ensuite filtrée par voie stérile et chargée dans un flacon ou une cartouche pour son utilisation, dans lequel l'excipient comprend un agent de stabilisation polyol, et dans lequel ladite composition comprend en outre un conservateur acceptable pour l'administration par voie parentérale.

2. Procédé selon la revendication 1, dans lequel ledit agent tampon est sélectionné parmi le citrate, le tartrate ou l'acétate.

3. Procédé selon la revendication 2, dans lequel ledit agent tampon est l'acétate.

4. Procédé selon l'une quelconque des revendications 1 à 3, dans lequel la concentration dudit agent tampon dans ladite composition se situe dans la plage de 2 mM à 500 mM.

5. Procédé selon l'une quelconque des revendications 1 à 4, dans lequel ledit agent tampon maintient un pH de 3 à 6.

6. Procédé selon l'une quelconque des revendications 1 à 5, dans lequel ledit agent de stabilisation polyol est un saccharide, un alcool de sucre tel que le mannitol ou un alcool polyhydrique.

7. Procédé selon la revendication 6, dans lequel ledit agent de stabilisation polyol est présent en une quantité de 1 à 20 % en poids de la composition.

8. Procédé selon l'une quelconque des revendications précédentes, dans lequel ledit conservateur est le m-crésol ou l'alcool de benzoyle.

9. Procédé selon l'une quelconque des revendications 1 à 8, dans lequel ledit conservateur est présent en une quantité de 0,1 à 2 % en poids de la composition.

10. Procédé selon l'une quelconque des revendications 1 à 9, dans lequel la concentration de ladite PTH(1-34) se situe dans la plage de 100 µg/mL à 500 µg/mL.

11. Composition pharmaceutique se présentant sous la forme d'une solution stabilisée, comprenant : (a) une quantité thérapeutiquement efficace de PTH(1-34) humaine ; (b) une quantité efficace d'un agent de stabilisation polyol ; (c) un agent tampon en une quantité suffisante pour maintenir le pH de la composition dans une plage d'environ 3 à 7 ; (d) un conservateur acceptable pour l'administration par voie parentérale ; et (e) le reste étant de l'eau.
